(19) Europäisches Patentamt / European Patent Office / Office européen des brevets

(11) **EP 4 333 784 B1**

(12) **EUROPEAN PATENT SPECIFICATION**

(45) Date of publication and mention
of the grant of the patent:
**22.10.2025 Bulletin 2025/43**

(21) Application number: **22725081.8**

(22) Date of filing: **04.05.2022**

(51) International Patent Classification (IPC):
***A61F 13/15*** *(2006.01)*  ***A61F 13/494*** *(2006.01)*
***A61F 13/496*** *(2006.01)*

(52) Cooperative Patent Classification (CPC):
**A61F 13/15268; A61F 13/49406; A61F 13/496**

(86) International application number:
**PCT/US2022/027586**

(87) International publication number:
**WO 2022/235734 (10.11.2022 Gazette 2022/45)**

(54) **DURABLE ABSORBENT PANT WITH IMPROVED ABSORBENT GUSSET CONFIGURATION**

HALTBARE ABSORBIERENDE HOSE MIT VERBESSERTER
ABSORPTIONSSEITENFALTENKONFIGURATION

CULOTTE ABSORBANTE DURABLE AVEC CONFIGURATION AMÉLIORÉE DE GOUSSET
ABSORBANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.05.2021 US 202163184839 P**

(43) Date of publication of application:
**13.03.2024 Bulletin 2024/11**

(73) Proprietor: **The Procter & Gamble Company
Cincinnati, OH 45202 (US)**

(72) Inventors:
• **SEPELLO, Cassandra, Ann
Cincinnati, Ohio 45202 (US)**
• **SEITZ, Bret, Darren
Cincinnati, Ohio 45202 (US)**

(74) Representative: **P&G Patent Germany
Procter & Gamble Service GmbH
Sulzbacher Straße 40
65824 Schwalbach am Taunus (DE)**

(56) References cited:
**CN-A- 110 022 816    US-A1- 2021 330 513**

**Description**

BACKGROUND

Disposable absorbent pants for managing urinary incontinence or menstrual flow have been marketed for a number of years. Many of these products have exhibited varying degrees of similarities in appearance, feel and bulkiness to disposable diapers or disposable children's training pants.

Finding these similarities undesirable, many people experiencing only mild to moderate incontinence have preferred not to use these products, opting to use durable/washable absorbent underwear. Additionally, some market segments have exhibited a preference for durable absorbent underwear as an alternative to disposable sanitary napkins or disposable menstrual pants, for various reasons including comfort, appearance (*e.g.*, discreetness under clothing), concerns for added burdens on waste management systems, concerns for raw material sourcing sustainability, etc.

CN110022816B relates to absorbent articles whose chassis comprises a plurality of elastics configured to deliver low pressure under the elastics.

In order for a durable absorbent underwear pant product to satisfactorily meet the needs of most users/wearers, ideally, it should: be comfortable to wear; be discreet under outer clothing; be effective at containment and absorption of exudates; have sufficient absorption capacity for a reasonable duration of wear; and be effective at preventing absorbed fluid from being expressed and leaked from the absorbent structure following absorption. It will be appreciated that a single product cannot perfectly meet all of these objectives, because they present conflicting demands on the product configuration. Nevertheless, there is room for improvement in existing configurations.

BRIEF DESCRIPTION OF THE DRAWINGS

FIG. 1 is a simplified depiction of an example of a brief pant, as it would appear laid out flat on a horizontal planar surface, front waist portion facing up.

FIG. 2 is a depiction of an example of the brief pant of FIG. 1, in an opened configuration wherein the front and rear waist portions have been separated at the hip portions or hip side seams, as it would appear laid out flat on a horizontal planar surface, wearer-facing surfaces facing up.

FIGS. 3A-3F are schematic lateral cross sections through a portion of a gusset including a leg edge, taken along line 3-3 in FIG. 2, for a brief pant, in various alternative examples.

FIG. 4 is a schematic longitudinal cross section through a portion of a gusset including a forward seam, taken along line 4-4 in FIG. 2, for a brief pant in one example.

FIG. 5A is a simplified depiction of an example of a brief pant, as it would appear laid out flat on a horizontal planar surface, front waist portion facing up.

FIG. 5B is a simplified depiction of an example of a shorts pant (or legged pant), as it would appear laid out flat on a horizontal planar surface, front waist portion facing up.

FIG. 6 depicts plots of stress (y-axis) vs. strain (x-axis) measured for crotch gussets of various examples of brief pants.

FIG. 7A is a plan view of an example of a crotch gusset laid out on a horizontal surface, wearer-facing surface facing the viewer.

FIG. 7B is a schematic vertical cross section of a portion of the gusset shown in FIG. 7A.

Definitions

With respect to a wearable garment such as a pant, "durable" means made predominately of cloth material that is knitted and/or woven from natural, semi-synthetic or synthetic fiber, thread or yarn, and which may be normally laundered or hand-washed and dried for reuse/re-wear a plurality of times without substantial loss of original shape, structure or useful mechanical attributes.

As used herein, "fabric" means a web material that is knitted or woven of fibers, or threads or yarns of fibers, and does not include a "film" or a fibrous nonwoven web material.

As used herein, "film" means a skin- or membrane-like material that is cast, extruded or formed in place, from a molten thermoplastic material. "Film" does not include fibrous nonwoven web material or fabric.

With respect to a pant in an opened configuration, laid out flat on a horizontal planar surface, "longitudinal" refers to a direction generally perpendicular to a line tangent each of the left and right leg opening edges where they are closest the front waist edge. With respect to a pant in an assembled configuration, laid out flat on a horizontal planar surface, front waist portion facing up, "longitudinal" refers to a direction generally perpendicular to a line tangent each of the left and right leg opening edges where they are closest the front waist edge. "Length" refers to a dimension measured along the longitudinal direction.

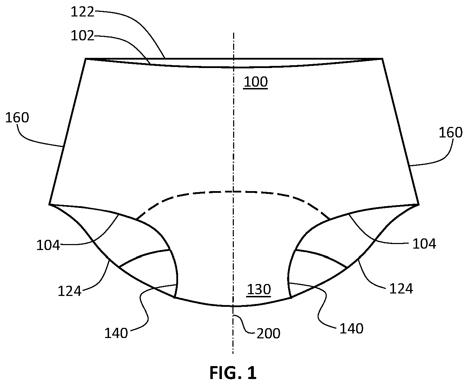

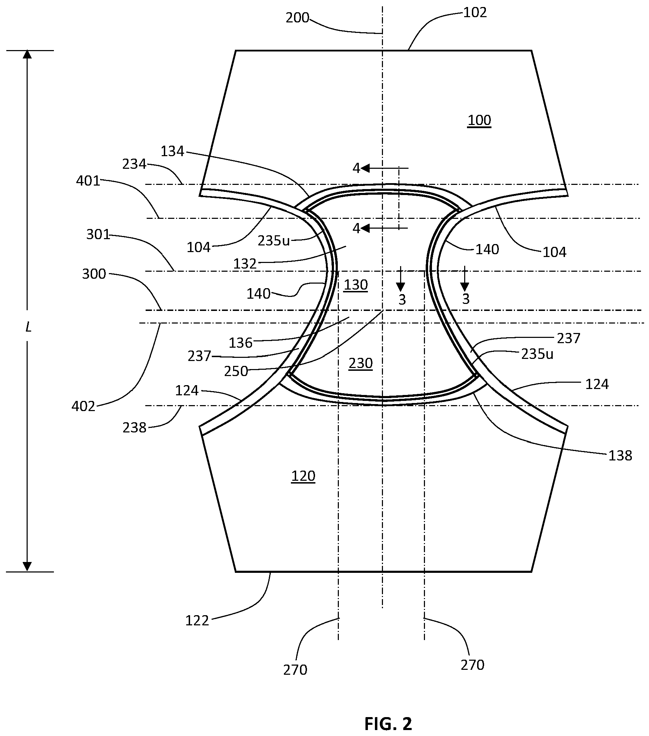

With respect to a pant in an opened configuration (*e.g.*, as illustrated in FIG. 2), laid out flat on a horizontal planar surface, "lateral" refers to a direction generally parallel to a line tangent each of the left and right leg opening edges where they are closest the front waist edge. With respect to a pant in an assembled configuration (*e.g.*, as illustrated in FIG. 1), laid out flat on a horizontal planar surface, front waist portion facing up, "lateral" refers to a direction generally parallel to a line tangent each of the left and right leg opening edges where they are closest the front waist edge. "Width" refers to a dimension measured along the lateral direction.

With respect to a pant in an opened configuration (*e.g.*, as illustrated in FIG. 2), laid out flat on a horizontal planar surface, the "z-direction" is the direction orthogonal to the longitudinal and lateral directions, *i.e.,* the vertical direction relative the horizontal planar surface.

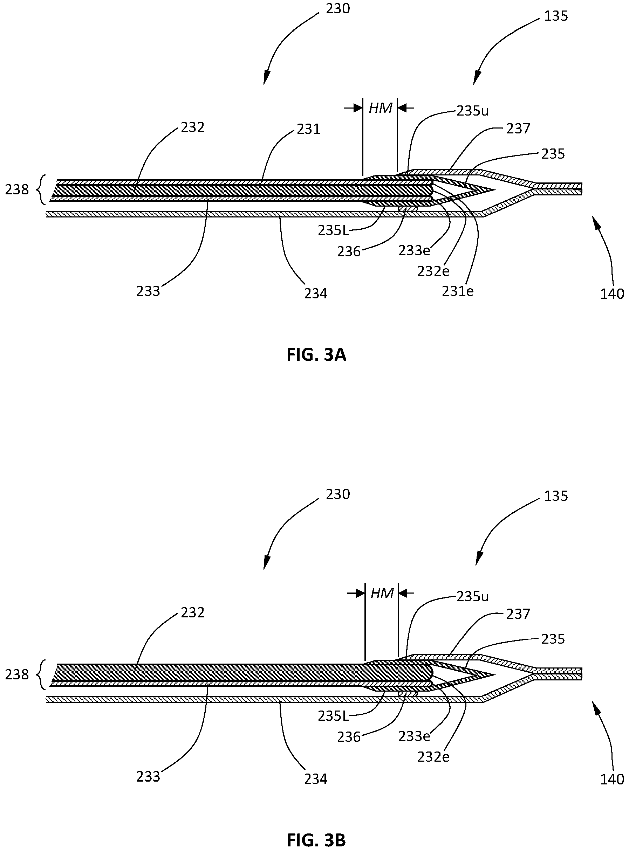

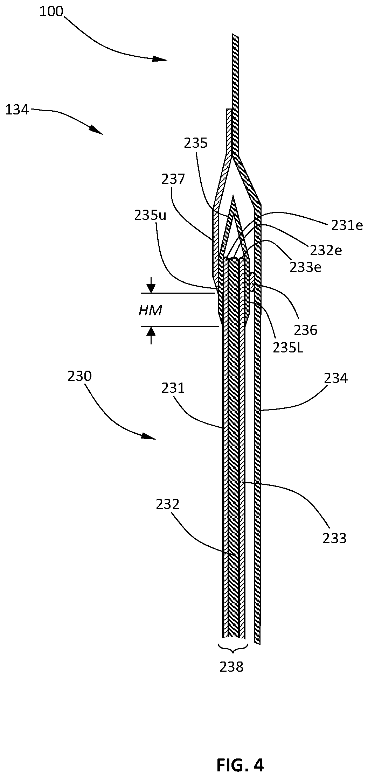

With respect to respective layer components in a crotch gusset of a pant in an opened configuration (*e.g.*, as illustrated in FIG. 2), laid out flat on a horizontal planar surface, wearer-facing surfaces facing up, as between first and second layer components in the crotch portion, the terms "above," "upper," "superadjacent," "below," "lower," "subjacent" and/or "beneath" describe the components' disposition along the z-direction relative each other. Thus, for example, referring to FIGS. 3A and 4, a wearer-facing layer 231 is disposed "above" an outward-facing layer 234, and conversely, the outward-facing layer 234 is disposed "below" the wearer-facing layer 231 and/or absorbent layer 232. "Superadjacent" and "subjacent" with respect to two layer components, mean further, that the two layer components are disposed in direct surface-to-surface contact with each other.

A yarn, thread, fiber, filament, web, film or fabric material, or a laminate or composite of any of these, is considered to be "elastically extensible" for purposes herein if, when a tensile force no greater than 50 gf/mm (gf per mm of sample width, where width is measured perpendicular to the stretch direction) is applied to the subject material along a stretch direction, the material may be extended along the direction to an elongated dimension of at least 130% of its original relaxed dimension (*i.e.,* can extend at least 30%), without rupture or breakage which substantially damages the subject material; and when the force is removed from the subject material, the material retracts along the stretch direction to recover at least 40% of such elongation. To illustrate, if a section of fabric having an original relaxed length of 100 mm and a width of 40 mm can be elongated by tensile force of 2000 gf (50 gf/mm) in a direction along its length to 130 mm length without substantial damage, and will retract upon removal of the force to a length no greater than 118 mm (130 mm - 118 mm = 12 mm = 40% of 30 mm), it is "elastically extensible" as defined herein. "Elongation," used herein to quantify and express an amount of strain imparted to an elastically extensible material in a stretch direction, means: {[(strained length of the strand) - (length of the strand before straining)] / (length of the strand before straining)}, $\times$ 100%. For a monolithic material such as a monolithic film material, "elastomeric" means "elastically extensible" as defined herein.

With respect to two opposing surfaces of a layer component of a pant, or combination of layer components, "wearer-facing" refers to the surface that faces the wearer's skin when the pant is worn normally; and "outward-facing" refers to the surface that faces away from the wearer's skin. With respect to two distinct layered components of a pant, the "wearer-facing" component is the component that is disposed closest the wearer's skin when the pant is worn normally; and the "outward-facing" component is the component that is disposed farthest from the wearer's skin.

For purposes herein, "pant" includes any garment adapted for wear about the human lower torso, including a front waist portion and a rear waist portion that join about the wearer's hips and beneath the wearer's crotch, to form a garment having a waist opening and a pair of leg openings. Herein, the term "pant" encompasses (but is not limited to) a garment defined herein as a "brief pant"; a garment defined herein as a "legged pant", and any other garment whether adapted for use as underwear or outerwear, having such features.

For purposes herein, unless otherwise specified, with respect to the proportionate content of a component material in a combination or structure, "predominate" means the component constitutes the majority of the weight of the combination or structure.

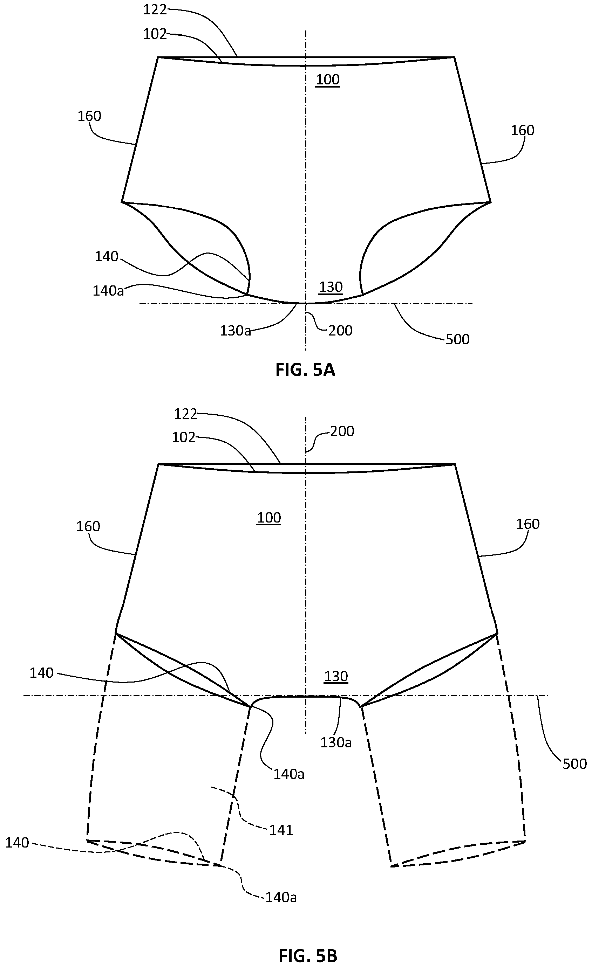

For purposes herein, a "brief pant" is distinguished from a "legged pant" by the configuration of the inside leg edges, resulting from the manner in which the component materials are shaped, sized, proportioned and seamed or otherwise affixed together. FIG. 5A depicts an example of a brief pant and FIG. 5B depicts an example of a legged pant in alternative forms. When the garment in its assembled condition is laid out flat on a horizontal planar surface, front waist portion facing up, and a lateral crotch tangent line 500 is drawn perpendicularly to longitudinal axis 200 and tangent to the point at which the crotch portion lower profile 130a intersects the longitudinal axis 200,

for a brief pant, the lowermost points 140a along the inside leg opening edges 140 are disposed along or above the crotch tangent line 500 (*i.e.,* toward the front waist edge 102) (see FIG. 5A); and
For a legged pant, the lowermost points 140a along the inside leg opening edges 140 are disposed below the crotch tangent line 500 (*i.e.,* away from the front waist edge 102) (see FIG. 5B).

As reflected in FIG. 5B, for purposes herein, a legged pant may have leg portions 141 of any length, wherein the lowermost points 140a of inside leg opening edges 140 are disposed below the crotch tangent line 500. Underwear shorts pants such as "boy short" styles will generally have shorter leg portions as suggested in the solid-line portions of FIG. 5B, while other legged pants may have leg portions of varying lengths, as suggested by the dashed-line portions of FIG. 5B.

Description of Embodiments

One problem presented in designing an absorbent system to be used to contain and absorb unintended (incontinent) small discharges of urine by a woman results from adhering urine flow. As a result of relatively low velocity of unintended discharges, female anatomical features, and typical surface chemistry of human skin (which can cause skin surfaces to attract and be relatively wettable by aqueous solutions such as urine), adhering flow has always been a phenomenon associated with low to moderate adult female incontinence. If features of a chosen containment/absorbency system do not reliably hold absorbent materials against the wearer's skin to intercept adhering flow, leakage can result.

It is believed, however, that a durable adult absorbent brief pant (*i.e.,* a pant with a crotch portion including an absorbent structure and a liquid impermeable barrier layer to the outside thereof), that reliably protects against leakage of adhering flow through a variety of body movements and positions and over a reasonable duration of wear/use has not been marketed to date. Currently marketed pant products do not effectively hold included absorbent structures against the body through various body movements, over a reasonable duration of wear/use. It is believed that the garment industry to date has failed to recognize that suitably oriented elastic stretch is important for maintaining proximity and/or contact of the absorbent structure with the wearer's body contours and skin surfaces through various body positions and movements, and that a structure having a combination of absorbent capacity suitable for providing protection against leakage from light to moderate incontinence, while having suitable directional elastic stretch so as to maintain effective contact of the structure with the wearer's body, may be designed. The human body and particularly the female lower torso and crotch region have geometrically non-ruled contoured surfaces. Fabrics or web materials that do not exhibit an effective amount of elastic stretch capability along an effective direction will not effectively conform closely to (*i.e.,* remain in contact with) the substantial majorities or entireties of these surfaces through ordinary body positions and movements. Increasing the amount of elastic stretch capability imparted to crotch gusset increases the variety of body shapes and movements to which the gusset can conform in use. In connection therewith, reducing the longitudinal tensile modulus (longitudinal tensile force in the material resulting from longitudinal stretching) tends also to increase body conformity and enhance comfort.

For underwear, many women prefer brief pants rather than legged pants such as "boy shorts" styles for ordinary daily wear. This is due to issues of comfort; unlike a legged, shorts-type pant, a brief pant ordinarily will not ride up and bunch about the legs from changes of body position, and thereby be a source of unwanted concentration of material bulk, tightness about the legs or other discomfort under outer clothing. Further, due to the manner in which their leg edges tend to cause the pant to fit through the crotch region of the body, brief pants having suitable elastic stretch characteristics may be preferred for maintaining a close fit about the female genital/urethra area, for purposes of protecting against leakage of unintended discharges of urine.

It has been learned that a durable absorbent pant may be designed that more reliably and comfortably holds an absorbent structure against/in contact with a wearer's body through the crotch region, in a position to better intercept adhering urine flow, capture the urine in the absorbent material before it can escape, and permit expansion of the absorbent components, initially and over a reasonable duration of wear/use of the pad. In order for a fabric or web of material to conform to a non-ruled surface, it must be capable of stretching along at least one direction. It has been determined that the direction of stretch that most effectively enables a fabric or web of a pant garment to effectively conform to the contours of the female lower torso and crotch region is, substantially, the longitudinal direction.

Referring to FIGS. 1 and 2 as an illustrative but non-limiting example, a pant may include a front waist portion 100, a rear waist portion 120 and a crotch portion 130 bridging the front and rear waist portions. Front waist portion 100 has a front waist edge 102, and left and right front leg opening edges 104. Rear waist portion 120 has a rear waist edge 122, and left and right rear leg opening edges 124. Crotch portion 130 has left and right crotch leg opening edges 140. Crotch portion 130 may include or consist of a crotch gusset 230 that may include several layer components that will be described further below. (For purposes herein, the "crotch portion 130" is a portion of a pant identified as described herein, independently of specific components or structures. A "crotch gusset 230" is a structural component that includes at least two distinct layers including an absorbent layer and a liquid impermeable barrier layer, and bridges the front waist portion 100 and the rear waist portion 120. A crotch gusset has a "liquid impermeable barrier layer" if it exhibits z-Direction Leakage no greater than 0.1 ml of test fluid into a section of filter paper, in the Liquid Impermeability test described below. Referring to FIG. 2 by way of example, for purposes herein, crotch portion 130 is the portion of the pant, at a minimum, lying between crotch portion minimum front extent 401 and crotch portion minimum rear extent 402, longitudinally centered about crotch portion lateral axis 301 (which is drawn along the smallest width dimension measured between the crotch leg opening edges 140), plus 10 percent of the overall length L of the pant to the front and the rear of the crotch portion lateral axis 301. As suggested by FIG. 2, it may be desired that the crotch portion lateral axis 301 be disposed forward of the lateral axis 300 (which equally divides overall length L), rather than be co-located with lateral axis 300, for purposes of better fit about an adult female wearer's legs and lower torso. Thus, the respective boundaries between crotch portion 130 and front and rear waist portions 100, 120 for purposes herein are independent of the location(s) of any seams such as seams 134, 138 that may be present to join material(s) included in the crotch gusset 230 and material(s) included in the front and rear waist portions 100, 120.

Material(s) forming one or both of forward and rearward portions 132, 136 of crotch portion 130 may be continuous with material(s) forming front and rear waist portions 100, 120, or alternatively, one or both of forward and rearward portions 132, 136 of crotch portion 130 and/or crotch gusset 230 may be substantially formed of one or more sections or layers of material that are distinct from material(s) substantially forming one or both of front and rear waist portions 100, 120, and crotch gusset 230 may be joined to front and rear waist portions 100, 120 at one or both of forward seam 134 and rearward seam 138. In an illustrative but non-limiting example reflected in FIGS. 1, 2 and 4, front waist portion 100, an outward-facing layer 234 of crotch gusset 230, and rear waist portion 120, may all be formed partially or entirely of a first single, continuous section of material, e.g., the fabric from which the main body of the pant is formed. In the example shown, one or more additional layers of material 231, 232, 233 may be added to the wearer-facing side of layer 234 as shown, and be connected or affixed directly or indirectly to the first section of material via, e.g., stitching/sewing, adhesive bonding, thermal bonding (fusing or welding) or other suitable attachment/joining mechanism (hereinafter, attachment mechanism) at, e.g., forward and rearward gusset seams 134, 138 and/or along the crotch leg opening edges 140.

In other non-limiting examples (not specifically shown), the sections of materials respectively forming front waist portion 100, rear waist portion 120 and crotch gusset 230 may be entirely separate and distinct, and joined via any suitable attachment mechanism at forward and rearward seams 134, 138. This configuration may be preferred in some circumstances because it may provide the designer with greater flexibility in selection of the respective materials for the waist and crotch portions with respect to appearance, feel, weight, breathability, elongation, stretch characteristics and cost. The elongation and stretch characteristics described below would be applicable to the one, or more layers of material in combination, present in crotch portion 130 and/or crotch gusset 230.

Crotch Portion / Crotch Gusset

Caliper

For purposes of minimized bulk, it may be desired that the combination of layered materials present in the crotch portion 130 and/or crotch gusset 230 be selected and configured so has to have a central z-direction Caliper not exceeding about 5 mm, more preferably not exceeding about 4 mm, and even more preferably not exceeding about 3.5 mm. (Herein, the "central z-direction Caliper" or "central Caliper" is measured at a location in the crotch portion 130 corresponding with the intersection 250 of the longitudinal 200 and lateral 300 axes of the pant, when opened and laid out flat on a horizontal planar surface.) Based on disclosure herein and teachings available in the art concerning textiles, persons of ordinary skill in the art will be equipped to select a combination of suitable materials to create a structure in the crotch portion 130 such as a crotch gusset 230 having such Caliper, along with other features and attributes described herein.

Absorption Capacity

In order for a durable absorbent pant to provide suitable absorbency and protection against leakage of unintended discharges of urine over a reasonable duration of wear, for a woman experiencing light to moderate incontinence, while balancing concerns for limiting caliper/bulkiness of the crotch portion while limiting the required relative planar size/surface area of an absorbent portion, it may be desired that a combination of material forming the structure within the crotch portion 130 of the pant have an Area Absorption Capacity from 0.1 ml/cm$^2$ to 0.4 ml/cm$^2$, and a Volume Absorption Capacity from 0.4 ml/cm$^3$ to 8.0 ml/cm$^3$, measured according to the Absorption Capacity Measurement method set forth below. Based on disclosure herein and teachings available in the art concerning textiles, persons of ordinary skill in the art will be equipped to select a combination of suitable materials to create a structure in the crotch portion 130 having such absorption capacity.

Elastic Stretch Attributes

Through experimentation it has been learned that imparting a pant with appropriate directional elastic stretch characteristics within the crotch portion 130 are important to provide a pant with a crotch portion that is both comfortably and securely held against the wearer's body surfaces, in position to intercept and absorb an adhering flow of urine, and thereby prevent leakage from the pant, through normal ranges of body positions and movements. It has been learned that the combination of materials present in the crotch portion 130 preferably should exhibit a maximum Longitudinal Elongation of 25 percent to 100 percent, measured according to the Maximum Elongation Measurement Method set forth below. Alternatively, or additionally, it may be desired that the combination of materials present in the crotch portion 130 exhibit a Longitudinal Tensile Modulus, measured according to the Longitudinal Tensile Modulus Method set forth below (reflecting the presence of one or more materials that impart elasticity) of 10 gf/mm to 100 gf/mm. Based on disclosure herein and teachings available in the art concerning textiles, persons of ordinary skill in the art will be equipped to select a combination of suitable materials to create a structure in the crotch portion 130 having such longitudinal elongation and tensile modulus properties.

Materials Selection

Generally, the front and rear waist portions 100, 120 of suitable examples of a pant may be formed of any fabric material or combination of fabric and other materials known and used as components of underwear, swimwear or athletic/active wear, exhibiting suitable attributes that may include, depending upon the location of the fabric within the structure, pleasing feel against the skin (softness and/or low-friction/smooth/silky feel), low caliper/bulk, elongation capability, elastic extensibility, absorbency, wicking ability, breathability, etc. In one example, the front and rear waist portions 100, 120, and the outward-facing layer 234 of the crotch gusset 230, may include or be formed of a knitted stretch fabric, in some examples, a combination of one or more of nylon, polyester, cotton and elastane fibers (e.g., Lycra spandex (a product of The Lycra Company, Wilmington, DE)).

Additional layer components of crotch portion 130 and/or crotch gusset 230, as herein described requiring absorbency and longitudinal elastic stretch attributes, may include a combination of several materials selected to impart the structure with the desired attributes.

Fabric Structure

Most durable fabrics exhibit anisotropic elongation capabilities.

Woven fabrics, formed by weaving, are formed of two groups (warp group and weft group) of interlaced constituent yarns or threads, the yarns or threads within each group being substantially parallel to each other, and substantially perpendicular to the yarns or threads in the other group, along the plane of the fabric. Unless the constituent yarns or threads are themselves formed of extensible material, woven materials have relatively low elongation capabilities along the warp and weft directions, and have relatively higher elongation capabilities along the two bias directions approximately 45 degrees from the warp and weft directions. Consequently, where it is desired for particular reasons that a layer component of the crotch portion 130 and/or crotch gusset 230 be a woven material, it may be desired that the material be oriented within the crotch portion such that one of the warp and weft directions is oriented from approximately 30 degrees to approximately 60 degrees, preferably from approximately 38 degrees to approximately 52 degrees, and more preferably approximately 45 degrees from the longitudinal direction of the pant, so as to provide maximum available longitudinal elongation capability for the woven layer.

However, when stretched along the bias direction, woven fabrics typically exhibit a substantial Poisson effect contraction along the trans-stretch direction (90 degrees from the stretch direction). When such a fabric is included in the crotch portion 130 with its bias oriented approximately along the longitudinal direction of the pant, the Poisson contraction effect may cause the crotch portion to laterally narrow, which may be deemed undesirable when effective coverage of the wearer's body through the crotch region is desired.

The constituent yarns or threads of knitted fabrics, by contrast, do not follow straight paths along the plane of the fabric, and are neither parallel nor perpendicular to each other. Rather, each constituent yarn or thread of a knitted fabric follows a looping path along successive rows, interlooping with one or more constituent yarns or threads in adjacent rows. As a consequence, knitted fabrics exhibit relatively greater elongation capability along all directions as compared with woven fabrics, even where the constituent yarns or threads themselves are not extensible. For this reason, unless a woven fabric is desired for a particular reason, it may be preferred that a knitted fabric be used to form any one or more, or all, of the fabric layers present in the crotch portion 130 and/or crotch gusset 230 of the pant.

Even so, most types of knitted fabrics have elongation capabilities that are anisotropic along the plane of the fabric, having a first direction of greatest elongation capability and a second direction, perpendicular to the first direction, of least elongation capability. Accordingly, when knitted fabric is selected and used to form one or more layers present in the crotch portion 130 and/or crotch gusset 230 of the pant, it may be desired that the fabric(s) forming any, some or all of the layers be oriented such that their directions of greatest elongation capability are at least approximately parallel with the longitudinal direction of the pant.

In some circumstances it may be desired that a knitted fabric selected to form a layer be a rib knit type. Rib knitted fabrics exhibit relatively high elongation capability along a direction parallel to the knit rows (perpendicular to the "ribs"), with relatively low Poisson contraction effect along the trans-stretch direction. Thus, in some circumstances, it may be desired that one or more layers present in the crotch portion 130 and/or crotch gusset 230 of the pant be formed of a rib knit fabric, with the "ribs" oriented substantially along the lateral direction of the pant.

In some circumstances it may be desired that the absorbent layer be formed of either woven or knitted terrycloth, for purposes of increasing aggregate fiber surface area and capillarity per unit fabric surface area, and thereby, providing increased absorbency to the absorbent layer 232, while still providing a durable fabric, in contrast to a nonwoven batt or matt of fibers. In conjunction therewith or as an alternative, and for purposes of enhancing absorbency, it may be desired that constituent fibers of the yarn(s) or thread(s) from which the absorbent layer fabric material is knitted or woven be in the form of microfibers (*i.e.,* fibers having an average denier of one (1) or less). It may be further desired that the constituent fibers be split microfibers. Yarns or threads formed of microfibers, particularly split microfibers, provide relatively greater fiber surface area per unit yarn/thread denier. When the fiber surfaces are hydrophilic, this imparts relatively greater absorbency to the fabric.

Fabrics Constituent Yarn/Thread Compositions

As contemplated herein, wearer-facing 231 and absorbent 232 layers present in the crotch portion and/or crotch gusset of the pant are expected to be exposed to discharges of urine and/or menstrual fluid, and are expected to receive, absorb and retain these fluids for a reasonable duration of wear time, preferably while leaving the wearer-facing surfaces as dry-feeling as possible. It may be desired that a wearer-facing layer 231 have a soft feel against the skin.

Accordingly, it may be desired that the wearer-facing layer be formed of a material that has a soft feel and has suitable wicking attributes so as to efficiently conduct discharged urine to an absorbent layer beneath, while having minimized tendency to retain urine and thereby have a wet feel for the wearer. Suitable materials may include polypropylene, polyesters and polyamides (e.g., nylon). Examples of these materials, when used to spin fiber components and/or when having received suitable hydrophilizing treatment, impart the spun fibers with suitable hydrophilic surface attributes (enhancing wicking), with relatively low individual fiber texture (reducing porosity and capillarity, and therefore, absorbency of the fabric). Additional materials may be incorporated in yarn or thread components for purposes of enhancing skin feel (e.g., enhancing a slick or silky feel against the skin) and/or further affecting hydrophilicity and/or reducing absorption tendencies. In some examples, polypropylene and/or polyethylene fiber components may be included for these purposes. In some examples, resins from which constituent fibers are spun may include additives to the primary polymer components, incorporated for enhancing skin feel, adjusting hydrophilicity, reducing absorbency, etc. In some examples, polyester or nylon component resins may include an additive comprising linolenic acid, to the extent of and as described in US 2017/0369698, for purposes of enhancing elongation and skin feel attributes, while reducing material usage and cost.

Although commercially processed cotton or other commercially processed natural fiber, or semi-synthetic, cellulose-derived materials such as rayon (for purposes herein, the term "rayon" includes rayon, viscose, lyocell, and any other fibers spun from reconstituted/regenerated cellulose), may be used or included as the component material for the absorbent layer for their inherent absorbency attributes, some of these materials may have a tendency, undesirably, to retain constituents of urine and/or other discharged fluids following laundering. Accordingly, one or more of polyester, polyamide and/or combinations thereof may be preferred as component resins or even the main/predominate component resins from which fiber components of yarn or thread components of fabrics for the absorbent layer 232 are formed. The constituent threads or yarns of the absorbent layer may be knitted in a manner that imparts a relatively lofty and/or densely fibrous network having a degree of void volume and capillarity making it suitable for absorbing fluid. In some examples, the knit may be a knitted terrycloth, or even a knitted/sheared terrycloth. The constituent threads or yarns may be or may include microfibers, for increased fiber surface area per unit x-y surface area of the absorbent assembly.

For purposes of imparting elastic extensibity to layers present in the crotch region (particularly longitudinal stretch), it may be desired that yarn or thread components of one or more of the fabrics present include elastically extensible fibers, yarns or threads. In some examples, elastically extensible fibers, yarns or threads may be formed of or include elastane or spandex (such as LYCRA, currently available from The LYCRA Company, Wilmington, Delaware), which are particularly elastically extensible and durable through a plurality of launderings, as compared to other elastic materials used to elasticize fabrics.

In other examples, one or more elastically extensible polymer film layers distinct from the fabric layer(s) in the crotch portion 130 may be included to impart elastic extensibility to the structure in the crotch portion as a whole.

An elastic polymer film layer may be formed of any suitable elastic polymer material. In some examples, an elastically extensible film layer may be formed by extrusion or other application of film resin in molten or semi-molten form onto a layer component fabric, whereby the molten resin partially penetrates the fabric and upon cooling forms a film that is partially mechanically enmeshed in and/or made integral with the fabric.

Antimicrobial Agents

For purposes of hindering growth of microorganisms supported by absorbed urine or menstrual fluid, which may cause odor, it may be desired to include one or more antimicrobial agents in or among the materials present in the crotch portion 130. Any such antimicrobial agents are preferably included in a form adapted to remain in place and continue to be effective following a plurality of launderings of the pant. In some examples, an antimicrobial agent may include particles or fibers including a metal, metal alloy or metallic compound that includes one or more of copper, silver, zinc, aluminum or combinations thereof. In other examples an antimicrobial agent may include particles or fibers including carbon or a composition or compound including carbon. One or more of these materials may be included as additives to resins from which constituent fibers are spun, or may be included in compositions that are topically applied to constituent yarns, threads or fabrics following manufacture thereof. Such antimicrobial agents are preferably included in material(s) forming the absorbent layer 232 and/or the wearer-facing layer 231.

Barrier Layer

Where urine or menstrual fluid is absorbed by a fabric layer in the crotch portion 130 and/or crotch gusset 230, it may be desirable to include a barrier layer, *e.g.,* barrier layer 233 (*see* FIGS. 3A-3D, 4) to prevent the absorbed fluid from passing from the absorbent layer to an outward-facing layer 234 or even to outer clothing. In some examples, a barrier layer 233 may be formed of or include a suitable liquid impermeable polymer film. In some examples, the film composition may be selected to have elastic extensibility, providing suitable elastic stretch attributes to the combination of layers present in the crotch portion 130 and/or crotch gusset 230. In some examples, a barrier layer 233 may comprise a film formed in whole or in part of a polyurethane- or polyester-based resin. In some examples, a barrier layer 233 may comprise a film that is formed by extrusion or other application of thermoplastic film resin in molten or semi-molten form directly onto the outward-facing surface of an overlying layer, such as absorbent layer 232, such that the film resin, while still molten, partially penetrates the fabric and thereby forms a liquid impermeable film that is partially mechanically enmeshed in and/or made integral with the fabric of the overlying layer. This also has the effects of consolidating these layers, which can reduce caliper in the crotch portion 130, and reducing or preventing wrinkling or bunching of the absorbent layer 232 upon elastic contraction. In a particular example, a polyurethane or polyester film may be formed by extrusion or other application of molten thermoplastic resin directly onto an outward-facing surface of a fabric that serves as or forms a component of absorbent layer 232. If the barrier layer 233 is separately formed, however, it may be desired to affixed it to the overlying layer, *e.g.* absorbent layer 232, via a suitable bonding mechanism, *e.g.,* an adhesive disposed between the barrier layer and the overlying layer. A configuration that allows the barrier layer and the overlying layer to separate, so as to result a void space between them, may be deemed undesirable because this may allow fluid to pool in the void space and thereby be uncontrolled and/or unmanaged by an absorbent structure, creating added risk of leakage.
The material selected for the barrier layer 233 may also be vapor permeable or "breathable" in that it can permit gas or water vapor to pass therethrough, while still being effectively liquid impermeable under ordinary conditions of the use contemplated herein, via a combination of having a porous structure for vapor permeability, but sufficiently small pore sizes and surfaces having low wettability (*e.g.* hydrophobic surfaces), for liquid impermeability. Various liquid impermeable, vapor permeable films and other materials are known and used in fields including personal hygiene and wound dressing applications. A liquid impermeable but vapor permeable barrier layer may be preferred in some circumstances for purposes of venting water vapor to improve wearer comfort and/or help avoid overhydration of the wearer's skin.
It some circumstances including those described below (wherein, for example, the barrier layer forms a part of an edge sealing structure), it may be desired that the material(s) selected to form the barrier layer 233 be, or be processed to result in, hydrophobic surfaces.

Other Construction Details

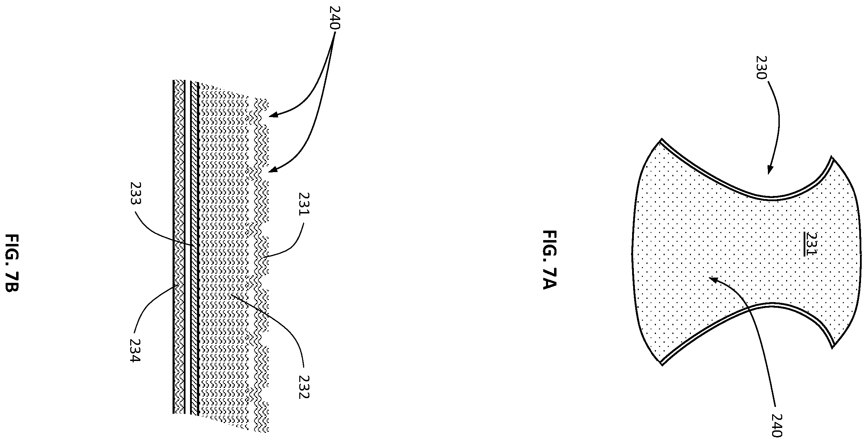

In some examples one or more layers within the crotch portion 130 and/or crotch gusset 230 may be bonded together via any suitable bonding mechanism. Referring to FIGS. 7A and 7B for example, a wearer-facing layer 231 may be bonded to a subjacent absorbent layer 232 for purposes of holding the layers in close proximity and thereby helping maintain a desired low caliper of the structure, and enhancing fluid communication between the layers, *i.e.,* enhancing the structure's ability to pass discharged urine from the wearer-facing layer to the underlying absorbent layer, via contact between the layers. In such example, the bonding mechanism should be selected so as not to occlude the interface between the two layers along any substantial portion of their interfacing surface areas. Thus, in some examples it may be desired that the bonding mechanism have the form of a discontinuous pattern of discrete bonds (*i.e.,* a pattern with unbonded regions between the bonds), such as a regular pattern of spot bonds 240. In some examples, bonds between the layers may be formed by discrete deposits of adhesive between the layers, adhering them together at the locations of the deposits. In other examples, bonds between the layers may be formed by thermal compression bonding. The latter bonding mechanism may be more durable through a plurality of launderings, and as illustrated schematically in FIG. 7B, creates a corresponding pattern of z-direction depressions in the wearer-facing layer 231 that may serve to collect discharged urine/fluid and initiate and/or facilitate its movement (via wicking) down to the absorbent layer 232. For purposes of enabling thermal compression bonding, the wearer-facing layer and the subjacent absorbent layer may each include polymer components of respective fusible compositions (such as similar polyester-based compositions) that facilitate formation of robust bonds between the layers upon localized application of heat and pressure at the bond sites. (Herein, "fusible compositions," with respect to two respective polymers present in two respective layers, means that the two polymers are miscible, capable of melting and mixing at a temperature of 250 °C or lower, to form a single thermodynamic phase.)

Various layers that may be included in crotch portion 130 and/or crotch gusset 230, e.g., layers 231, 232, 233 and 234, may also be joined to each other by any suitable mechanism at forward and rearward seams 134, 138 and crotch side seams 135 proximate the leg edges 140. The joining mechanism may be a system of stitching to affix the layers together; however, for purposes of liquid containment it may be desired that the joining mechanism include a generally hydrophobic, water insoluble adhesive or adhesive film, by itself or as a supplement to stitching.

Seam Construction

Now referring to FIGS. 2, 3A-3F and 4, through experimentation it has been learned that it may be desirable to incorporate the absorbent assembly 238 (including at least an absorbent layer 232, a barrier layer 233, and optionally including an additional, wearer-facing layer 231) into the pant structure, in a manner that minimizes potential for fluid to migrate from the absorbent structure laterally outwardly, or longitudinally upwardly, beyond any of seams 134, 135, 138. Fluid that is allowed to migrate past these seams can soil the fabric forming the pant in front or rear waist portions 100, 120, or even outside clothing, bedclothes, etc. Chances of such fluid migration may be substantially reduced by features described and depicted herein.

Given that a desirable feature of materials forming a wearer facing layer 231 and/or absorbent layer 232 may include the ability of these materials to wick fluid, features that prevent such wicking longitudinally and laterally outward, past seams 134, 135 and 138, may be desired.

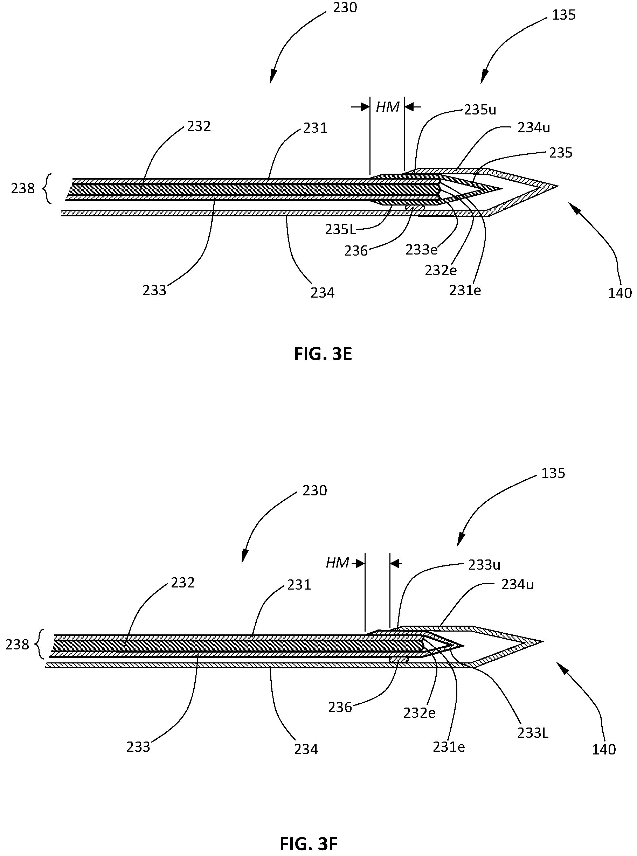

Referring to FIGS. 3A, 3B, 3E and 4, in one example, any or all of these seams may include an edge sealing structure including an edge sealing strip 235, wrapping about the absorbent assembly 238 at the periphery thereof, proximate any or all of seams 134, 135 and 138. Edge sealing strip 235 may include a strip of film that is liquid impermeable and hydrophobic. As depicted in the figures, the film may be included so as to be wrap from the bottom (outward-facing) side of barrier layer 233, about edges 233e, 232e and 231e (if included), and adhered to the wearer-facing surface of the wearer-facing layer of the absorbent assembly (such as wearer-facing layer 231, as in FIGS. 3A and 4, or absorbent layer 232 alone, as in FIG 3B). The film selected to form the edge sealing strip 235 may be a heat-activated adhesive film strip or tape, which may be adhered to the respective layers as shown via application of heat, e.g., in a heated press. Alternatively, the film selected to form the edge sealing strip 235 may be a liquid impermeable film strip or tape adhered to the respective layers as shown via an applied adhesive (not specifically shown). The edge sealing strip 235 may be formed of a single continuous/integral piece of material, or may be formed of two or more pieces of film material that are fused or adhered together, for example, an upper piece and a lower piece. The material forming all or part of edge sealing strip 235 may be selected to be an elastically extensible material, at least for those portions proximate the leg edges 140. This will help impart or retain elastic extensibility for the portions of the pant structure proximate the leg edges (e.g., leg bands).

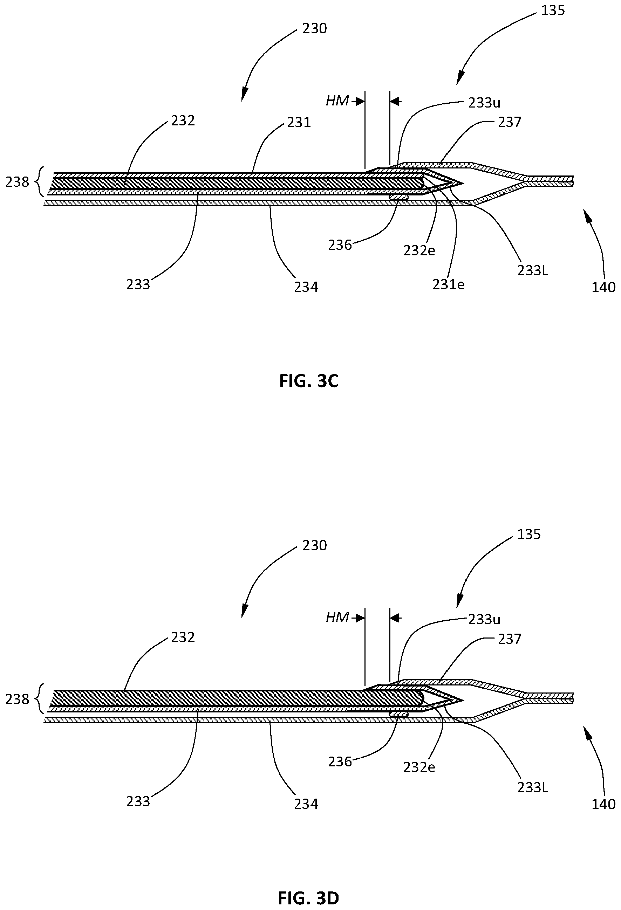

In another example, referring to FIGS. 3C, 3D and 3F, an edge sealing structure may be provided by extended margins of the liquid impermeable barrier layer 233, wrapped from the bottom of the absorbent layer, about edges 232e and 231e (if included) and over the wearer-facing surface of the absorbent assembly 238 as shown in the figures. The extended margins of the barrier layer 233 may be adhered to the wearer-facing surface of the assembly via heat activation, e.g., in a heated press (if the material of the barrier layer is thermoplastic/heat-activatable), or alternatively, via adhesive. As previously noted, the material of barrier layer 233 may be selected from among elastically extensible materials, thus imparting or retaining elastic extensibility for the portions of the pant structure proximate the leg edges (e.g., leg bands).

In addition to preventing fluid from wicking within the fabric layer(s) of the absorbent assembly longitudinally and laterally out past seams 134, 135 and/or 138, it may be desirable to include features that resist travel of unabsorbed fluid along the wearer-facing surfaces of the absorbent assembly, longitudinally and laterally out beyond any or all of these seams.

From FIGS. 3A-3F and 4, it can be seen that the material providing edge sealing may be extended longitudinally and/or laterally inwardly by a margin, inwardly beyond any overlying material that includes a wear-facing fabric (e.g. finishing material 237 or upper wrapping portion 234u of outward-facing layer 234). As shown in the figures, an upper portion 235u (FIGS. 3A, 3B, 3E and 4) or 233u (FIGS. 3C, 3D and 3F) may be sized to extend laterally and/or longitudinally inwardly (toward the center of the structure along the x-y plane) beyond overlying material 237 or 234u, by a barrier margin HM. Thus, upper portions 235u or 233u have upper, wearing-facing surfaces, uncovered by overlying material such as finishing material 237 or 234u. When the edge sealing film material forming upper portion 235u or 233u material is selected to have hydrophobic surfaces, the exposed surface resists traversal of aqueous fluid thereacross, from a more hydrophilic region (such as fabric of wearer-facing layer 231 and/or absorbent layer 232). Thus, traversal of urine or menstrual fluid deposited on the wearer-facing surface of the absorbent assembly 238 but remaining unabsorbed thereby, is resisted, helping to prevent soiling by fluid migrating along the wearer-facing surface, outwardly along an x-y direction past the seams.

As reflected in FIGS. 3A-3D, any or all of seams 134, 135 and 138 may be finished off on the wearer-facing sides thereof with a strip of finishing material 237, suitably selected for purposes of enhancing the appearance of the seam(s), enhancing wearer comfort, and unifying the materials at the leg edges to provide a finished appearance thereto. In one example, finishing material 237 may include a wearer-facing layer of a fabric material, selected for tactile softness/comfort against the wearer's skin. In a further example, finishing material 237 may be a laminate of a fabric on a wearer-facing side and a heat-activatable adhesive (not specifically shown) on an outward-facing side. The laminate may be applied and adhered to edge sealing strip 235 (*e.g.,* as shown in FIGS. 3A, 3B and 4) or edge-sealing portion(s) of barrier layer 233 (*e.g.,* as shown in FIGS. 3C and 3D), and to the wearer-facing side of outward-facing layer 234, via a heated press. In other examples, finishing material 237 may be applied and adhered to the edge sealing strip and to the outward facing layer by a separate adhesive applied between these materials. When finishing strip 237 includes a fabric in a wearer-facing position, the fabric may be a finely knitted, extensible fabric, and may be elastically extensible. If a layer of film and/or adhesive is included, it, too, may be selected to be elastically extensible, thereby imparting or retaining elastic extensibility for the seams, *e.g.*, proximate leg edges 140.

In other examples such as reflected in FIGS. 3E and 3F, seams 135 and 138 may be finished off on the wearer-facing sides by wrapping the fabric of the outward-facing layer 234 up and around leg opening edges to create wrapping portions 234u, wrapping over the edges of the absorbent assembly 238 proximate the leg openings. The wrapping portion 234u may be secured to the wearer-facing surface of the absorbent assembly via any suitable mechanism including compression and/or thermal bonding, stitching, or adhesive disposed between wrapping portion 234u and the absorbent assembly 238, although use of adhesive may be preferred to avoid puncturing or otherwise deforming or damaging (*e.g.*, puncturing by stitching) any of the layers of the structure at the risk of creating potential leakage pathways. Such other examples may provide an effective structure that in some circumstances may be more easily, more efficiently, and/or less expensively manufactured.

When finishing material 237 or a wrapping portion 234u is formed of or includes fabric as may be desired for purposes of appearance and/or wearer comfort, the fabric may have characteristics including a fibrous network that may cause it to wick aqueous fluid. Thus, it can be appreciated that including a barrier margin *HM* of exposed hydrophobic material, as described above, may be desired to resist flow of fluid along the wearer-facing surface of the pant from the absorbent assembly 238 to finishing material 237 or a wrapping portion 234u, where it could be further wicked out to, *e.g.,* the leg edges 140, and soil outer clothing, bedclothes, etc.

It may be desired that barrier margin *HM* is of a dimension sufficient to be greater than the size of a beaded droplet of urine and/or menstrual fluid. Thus, it may be desired that barrier margin *HM* be, on average, greater than 0 mm, more preferably at least 1 mm, and even more preferably at least 2 mm (as measured along any direction in the x-y plane normal to the edge profile of the outlying fabric at the measurement location).

To more securely unify the absorbent assembly 238 and the outward-facing layer 234, gusset adhesive 236 may be disposed between barrier layer 233 and outward-facing layer 234. In some examples, adhesive 236 may be disposed between lower portion 235L of edge sealing strip 235, and outward-facing layer 234 (*see, e.g.,* FIGS. 3A, 3B, 3E and 4). Adhesive 236 may be a heat-activated adhesive, activated to adhere the layers together via a heated press. In some examples it may be desired that adhesive 236 is predominately located proximate the seams 134, 135 and 138 as suggested in the figures, while portions or all of outward-facing layer 234 and barrier layer 233 between the seams remain unattached. This may help avoid wrinkling or bunching of the outward-facing layer during wearer movements, and/or otherwise provide or preserve a smooth, sleek appearance to the portions of the outward-facing layer underlying the absorbent assembly 238.

From the foregoing description and from the figures, it will be appreciated that, in the examples of structures described, x-y direction fluid migration, via wicking through fabric layers of the absorbent assembly, is stopped at the peripheral edges thereof by the edge sealing structure. Fluid migration in an x-y direction along the wearer-facing surface of the gusset is resisted by the film extending through the barrier margin *HM.* In this manner, fabric materials in the gusset subject to fluid deposition and fluid wicking are effectively isolated, or cut off from fluid communication, from surrounding fabric materials such as the outward-facing layer 234 and wrapping portion 234u thereof and/or fabric of the finishing material 237, along portions or all of the periphery of the gusset, proximate seams 134, 135 and 138.

It will be appreciated that FIGS. 3A-3F and 4 are only schematic depictions of arrangement of the various layer and seam components described herein, and that the voids suggested in the figures, above the outward-facing layer, within the edge sealing structure, and below the wearer-facing finishing material, would not necessarily be present - particularly when the pant structure is assembled and adhered together with the absorbent assembly via use of a press, which would press the layered materials along the z-direction into close face-to-face relationship, and compress any thermoplastic materials included (such as adhesives and thermoplastic film materials), thereby reducing or eliminating voids and reducing caliper in the gusset and at the seams 134, 135, 138.

Examples

Quantities of prototype brief pants were manufactured to include a crotch gusset, having the following layers, in order of wearer-facing (upper) layer to outward-facing (lower) layer:

- Wearer-facing layer: Knitted fabric, 66% polyester, 34% polypropylene; direction of greatest elongation oriented longitudinally;
- Absorbent layer: Knitted sheared pile terrycloth / sheared pile fabric, 86% polyester yarn, 14% nylon yarn; nylon yarn treated with antimicrobial agent including copper particles; direction of greatest elongation oriented longitudinally; and
- Barrier layer: Thermoplastic polyester elastomer (TPE-E) film, extruded onto outward-facing surface of absorbent layer; and
- Edge sealing strip: hydrophobic polyurethane-based film applied to entirely circumscribe and wrap the edges of the wearer-facing layer, absorbent layer and barrier layer, along the inside leg edges and forward and rearward seams.
- Outward-facing layer: knitted fabric, 76% nylon, 24% elastane.

For purposes of comparison, crotch gussets of examples of SPEAX (formerly ICON) brand absorbent brief pants (a product of Thinx inc., New York, New York), currently marketed to women experiencing incontinence, were measured and tested, according the methods described herein, for Maximum Longitudinal Elongation, Longitudinal Tensile Modulus, Area Absorption Capacity and Volume Absorption Capacity. For further comparison, crotch gussets of currently KNIX brand absorbent pants (product of Knix Wear Inc., Toronto, Ontario, Canada) and MODIBODI brand absorbent pants (a product of Modibodi, Balmain, AU and Nashville, TN, USA) (both currently marketed as menstrual pants). The results of the measurements/testing are set forth in the table below. (The comparison products did not include any containment barrier structures, but the crotch gussets were removed from the rest of the underwear keeping the bonding around the crotch portion intact, with the outer layer fabric being removed for the absorption capacity testing.)

| | SPEAX (marketed as incontinence pant) | KNIX (marketed as menstrual pant) | MODIBODI (marketed as menstrual pant) | Prototype |
|---|---|---|---|---|
| Crotch Width *CW* (cm) | 7.2 | 6.5 | 7.7 | 8.0 |
| Dry Central Caliper (mm) | 5.2 | 2.0 | 2.9 | 3.4 |
| Area Absorption Capacity (ml/cm$^2$) | 0.29 | 0.11 | 0.20 | 0.25 |
| Volume Absorption Capacity (ml/cm$^3$) | 0.565 | 0.517 | 0.690 | 0.727 |
| Maximum Longitudinal Elongation (percent) | 5.3 | 5.9 | 20.3 | 38.0 |
| Longitudinal Tensile Modulus (gf/mm) | 497.7 | 454.7 | 157.4 | 53.4 |

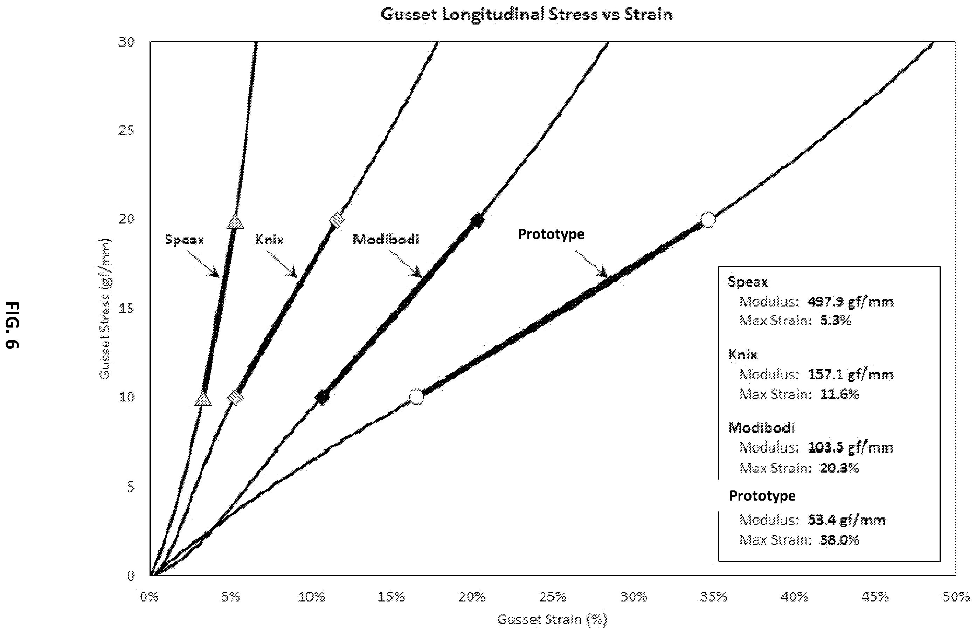

To graphically illustrate the differences between the elongation and tensile modulus characteristics of the prototypes as compared to examples of commercially available pants, the gusset longitudinal stress vs. strain are graphed in FIG. 6.

Measurement Methods

General Sample Preparation

Unless otherwise specified below, each of the measurements below is to be conducted on 10 separate like samples (taken from 10 separate like examples of pants) and the average of the 10 separate like samples is considered to be the measurement for that specific sample set.

Referring to FIG. 2, samples including the entire crotch gusset 230 are collected from examples of the subject pant. Lateral lines 234, 238 that are respectively tangent the forwardmost edge of the forward seam 134 and rearwardmost edge of the rearward seam 138 are identified, and the pant is cut apart along these lines (without cutting into the seam itself) to provide a sample that includes the entire crotch gusset 230.

If the pant is a legged pant, cut out the gusset in its entirety, along cutting paths outside of the seam(s) joining the gusset to the remainder of the pant, without cutting into the seams themselves. For legged pants, measurements of "Crotch Width" made for purposes herein will be the width of the removed gusset measured along the lateral direction, along a lateral line marking the shortest distance between the leg openings prior to removal of the gusset from the legged pant.

The sample should be cut from the example pant with a sharp knife or suitably sharp cutting device effective to precisely and cleanly cut the sample. A straight edge or other suitable drafting/drawing tool may be used where helpful to hold the example down on the work surface and help guide the cutting device.

The testing is performed under ambient room conditions (temperatures from between 15°C to 35°C and relative humidity from between 35% to 75%). Samples are conditioned for at least two hours prior to testing under the same conditions.

All linear dimensions are measured manually by ruler within the ordinary x-y plane, using a ruler that is traceable to NIST or other standards organization.

Longitudinal Tensile Modulus

For the Longitudinal Tensile Modulus and Maximum Longitudinal Elongation test methods, the samples as described above in "General Sample Preparation" are further modified by cutting out a 40 mm laterally wide section symmetrically about the longitudinal axis 200. The leg edge seams are not included in these tests. If the lateral width between the narrowest separation of leg edge seams as defined by the tangent lines 270 in FIG. 2 is less than 40 mm, then the pant is not deemed to be suitable (as being too narrow through the crotch region for suitable coverage) for purposes described herein, and is considered to be outside the scope of the claims.

The Longitudinal Tensile Modulus of the sample is determined by stretching along the direction of the longitudinal axis 200 of the pant, using a constant rate of extension tensile testing machine with computer interface, e.g., Instron; MTS; Zwick; etc., using a load cell for which the loads measured are within 10% to 90% of the limit of the cell, and ensures accuracy of a 5N load to 0.1N. The instrument is equipped with a single line contact grips, 8 cm in grip width. Prior to testing, calibrate the equipment according to the instruments manufacturer's recommendations.

In accordance with the sample preparation instructions set forth above, the Sample Width is 40 mm. The stress in the sample is calculated by dividing the force in the load cell by the Sample Width, and is expressed in units of gf (grams-force)/mm. (The caliper of the sample is not a factor in this calculation.) The Sample Length is equal to the length of the sample along the longitudinal axis 200 between the lateral lines 234, 238 (along which the sample was cut from the example pant), as illustrated by way of example in FIG. 2.

The grips of the tensile testing machine consist of air actuated grips designed to hold the sample. No slippage should be permitted between the sample and the grips. The distance between the grips (along the axis of the machine's elongation) should be the Sample Length minus 6 cm. This distance will be hereinafter referred to as the "Starting Gauge-Length".

The sample is mounted in the grips with its longitudinal axis 200 parallel to the direction of applied elongation, and centered in each grip. Two (2) cm of the sample's length at each end is inserted into each grip. The Starting Gauge Length, determined as described above, will ensure that 2 cm of longitudinal slack will be present in the sample at the start of the test.

After the sample is mounted, the machine's load channel is set to zero (this eliminates the weight of the sample in the calculations). The grips are slowly moved apart at 5.08 cm/min (2.0 in/min) until a load of 5 gf (grams-force) is reached. The separation between the grips at this position is recorded as L0.

$$(L0 = \text{Starting Gauge Length} + \text{additional machine extension to reach 5 gf})$$

After the 5 gf load is reached, extend the sample at a rate of 50.8 cm/min (20 in/min) with a data acquisition rate of 50 Hz. Extend until either a stress of 30 gf/mm is reached, or the sample breaks.

Sample strain is calculated by $\Delta L/L0$. $\Delta L$ is any additional extension between the grips after L0 is reached and is recorded along with load at a rate of 50 Hz. Sample strain is expressed numerically (not as a percentage), thus a strain of 100% is 1.0 for the purposes of these calculations.

Record the sample strains at sample stresses of 10 gf/mm and at 20 gf/mm.

Longitudinal Tensile Modulus is the linear slope between 10 gf/mm and 20 gf/mm, and is calculated as:

Longitudinal Tensile Modulus = [20 gf/mm - 10 gf/mm]/[sample strain at 20 gf/mm - sample strain at 10 gf/mm]

Repeat for 10 samples.

Maximum Longitudinal Elongation

Maximum Longitudinal Elongation is measured during the Longitudinal Tensile Modulus test. The Maximum Longitudinal Elongation is the sample strain at a sample stress of 20 gf/mm. Maximum Longitudinal Elongation is expressed as a percent strain, e.g., a value of 1.0 strain from the Longitudinal Tensile Modulus method is expressed as 100% strain for Elongation.

Repeat and record the results for 10 samples. Calculate and record the average of the results. The average will be the Maximum Longitudinal Elongation value for the subject pant design.

Absorption Capacity

The absorption capacity test measures the amount of liquid held within a test sample after specified times of immersion and vertical drainage. The amount of test liquid that is retained by the test sample is used to calculate and report the Area Absorption Capacity (milliliters (ml) of liquid per specimen area in square centimeters) and the Volume Absorption Capacity (in milliliters (ml) of liquid per specimen volume in cubic centimeters). All testing is performed in a room controlled at 23°C $\pm$ 3C° and 50% $\pm$ 2% relative humidity.

The test procedure follows compendial method WSP 010.1.R3 (12) part B (Liquid Absorptive Capacity) with modifications specified as follows. The test liquid is deionized water at room temperature (23°C $\pm$ 3C°; density 1.00 g/ml). For the weighing portions of the test, no cover glass is used as the test liquid is non-volatile. The overall dimensions of the wire gauze test specimen support is large enough (*e.g.* 12 inches by 12 inches) to accommodate the larger test sample size. The test sample is the entire gusset (as described herein), thus larger than what is suggested in the compendial method.

Prior to measuring absorption capacity, the examples of the pants of interest (prior to removal of samples therefrom) are washed in order to mimic in-use conditions and to follow the recommended "prior to use" instructions that accompany these types of pants (*e.g.* wash before use). The examples are placed into a mesh lingerie bag, and then placed into a high efficiency, front-loading washing machine (any convenient source) along with a single small/light load dosage of TIDE brand laundry detergent ("Original" designation; "HE" or other high efficiency washing machine designation; without additives such as FEBREZE, ODOR DEFENSE, OXI additives, bleach or bleaching additives or fabric softening additives) (product of The Procter & Gamble Company, Cincinnati, Ohio). The washer is set to delicate cycle using cold water. After the wash cycle, the examples are removed from the mesh bag and placed flat on a drying rack to air dry for about 12 hours. After air-drying, the examples are placed into a clothes dryer (any convenient source) set on the delicate cycle with very low heat for about 5 minutes or until dry to the touch.

Test samples are prepared as follows. The pre-washed and dried examples are equilibrated in a room controlled at 23°C $\pm$ 3C° and 50% $\pm$ 2% relative humidity for about 2 hours. Test samples that include the entire gusset are removed from the examples as described in the General Sample Preparation section herein. Using scissors, the outermost fabric layer on the outward-facing side of the gusset is cut out along the entire gusset shape, inboard of the seams, using care so as not to cut into any of the edge seams present.

The immersion and drainage procedure outlined in the compendial method is then followed with the modifications previously noted. Subtract the Dry Mass from the Wet Mass and record as Liquid Mass Absorbed to the nearest 0.01 grams. Since the density of deionized water is 1.00 g/ml, the Liquid Mass Absorbed is also recorded as Liquid Volume Absorbed to the nearest 0.01 ml. Divide the Liquid Volume Absorbed (ml) by the overall area (cm$^2$) of the test specimen and record as Area Absorption Capacity to the nearest 0.01 ml/cm$^2$. Now divide the Liquid Volume Absorbed (ml) by the volume of the test specimen (area $\times$ central caliper) and record as Volume Absorption Capacity to the nearest 0.01 ml/cm$^3$.

In like fashion, repeat for a total of three replicate test specimens. Calculate the arithmetic mean for Area Absorption Capacity and Volume Absorption Capacity and report to the nearest 0.01 ml/cm$^2$ and 0.01 ml/cm$^3$, respectively.

Caliper

The Caliper of a sample including a crotch gusset is measured as the distance between a reference platform on which the sample rests and a pressure foot that exerts a specified amount of pressure onto the sample over a specified amount of time. All measurements are performed in a laboratory maintained at 23 °C $\pm$ 2 C° and 50% $\pm$ 2% relative humidity.

Caliper is measured with a manually-operated micrometer equipped with a pressure foot capable of exerting a steady pressure of 0.5 kPa $\pm$ 0.01 kPa onto the test sample. The manually-operated micrometer is a dead-weight type instrument with readings accurate to 0.001 mm. A suitable instrument is Mitutoyo Series 543 ID-C Digimatic, available from VWR International, or equivalent. The pressure foot is a flat ground circular movable face with a diameter of 50 mm. The sample is supported by a horizontal flat reference platform that is larger than and parallel to the surface of the pressure foot. The system is calibrated and operated per the manufacturer's instructions.

Obtain a sample including the gusset by removing it from the pant, as described above. When excising the sample from an absorbent article, use care to not impart any wrinkles into the layers or other distortion of the layers during the removal process. Samples are conditioned at 23 °C ± 2 C° and 50% ± 2% relative humidity for 2 hours prior to testing. To measure caliper, first zero the micrometer against the horizontal flat reference platform. Place the sample on the platform with the desired measurement location centered below the pressure foot. Gently lower the pressure foot with a descent rate of 1.0 mm ± 0.1 mm per second until the full pressure is exerted on the sample. Wait 5 seconds and then record the caliper of the sample to the nearest 0.01 mm. In like fashion, repeat for a total of 10 replicate samples. Calculate the arithmetic mean for the Caliper and report to the nearest 0.01 mm.

Liquid Impermeability

When there is a question concerning whether a particular pant has a gusset with a liquid impermeable barrier layer, this Liquid Impermeability test method may be used to measure a quantity of test liquid that will pass through a sample and enable determination whether there is a "liquid impermeable barrier layer" present in the gusset, according to the definition set forth in the description above.

The Liquid Impermeability test measures the quantity of liquid transferred through to the outward-facing side of a test specimen obtained from a pant after it is dosed with a prescribed volume of test liquid in order to simulate a liquid insult during actual use/wear of the pant.

All testing is performed in a room controlled at 23°C ± 3C° and 50% ± 2% relative humidity.

Prior to performing the measurement of this method, the examples of the pants of interest (prior to removal of samples therefrom) are washed in order to mimic in-use conditions and to follow the recommended "prior to use" instructions that accompany these types of pants (*e.g.* wash before use). The examples are placed into a mesh lingerie bag, and then placed into a high efficiency, front-loading washing machine (any convenient source) along with a single small/light load dosage of TIDE brand laundry detergent ("Original" designation; "HE" or other high efficiency washing machine designation; without additives such as FEBREZE, ODOR DEFENSE, OXI additives, bleach or bleaching additives or fabric softening additives) (product of The Procter & Gamble Company, Cincinnati, Ohio), or equivalent. The washer is set to delicate cycle using cold water. After the wash cycle, the examples are removed from the mesh bag and placed flat on a drying rack to air dry for about 12 hours. After air-drying, the examples are placed into a clothes dryer (any convenient source) set on the delicate cycle with very low heat for about 5 minutes or until dry to the touch.

Test samples are prepared as follows. The pre-washed and dried example pants are equilibrated in a room controlled at 23°C ± 3C° and 50% ± 2% relative humidity for about 2 hours. Test samples containing the entire gusset are removed from the examples as described in the General Sample Preparation section herein. Using scissors, the outermost fabric layer on the outward-facing side of the gusset is cut out along the entire gusset shape, inboard of the seams, using care so as not to cut into any of the edge seams present. Mark the dose location at the intersection of the midpoint of the longitudinal axis of the sample and a lateral axis positioned at the narrowest portion of the specimen.

For each test sample, a single layer of filter paper is cut to 10 cm by 2.54 cm. A suitable filter paper is Ahlstrom Grade 989 (available from Ahlstrom-Munksjo North America LLC, Alpharetta, GA), or equivalent. The test liquid is deionized water at room temperature (23°C ± 3C°).

Record the mass of one layer of pre-cut filter paper and record as Dry Mass$_{fp}$ to the nearest 0.0001 grams. Place the pre-weighed filter paper onto a flat horizontal work surface. Position the test specimen centered over the filter paper with the garment facing side of the specimen facing the paper. Using a volumetric pipette, apply a 1.0 ml dose of test liquid to the pre-marked dosing location as follows. The tip of the pipette is held about 3 mm above the surface of the test specimen, and the dose is applied slowly (about 30 seconds) to avoid splashing. As soon as the entire dose has been applied, start a 1 minute timer. After 1 minute has elapsed, remove the test specimen and record the mass of the filter paper as Wet Mass$_{fp}$ to the nearest 0.0001 grams. Subtract the Dry Mass$_{fp}$ from the Wet Mass$_{fp}$ and record as z-Direction Leakage to the nearest 0.0001 grams.

In like fashion, repeat for a total of three replicate test specimens. Calculate the arithmetic mean for z-Direction Leakage and report to the nearest 0.0001 g.

Ordinary X-Y Plane Dimensions

For purposes herein, when a length or width of a feature of a pant is specified, it is to be measured with the pant laid out flat on a horizontal planar surface (in an opened or assembled configuration, as appropriate) with the material of the pant smoothed out flat, but in a relaxed condition, not pulled or stretched along any planar direction.

**Claims**

1.  A durable absorbent pant, comprising:

    a front waist portion (100) with a front waist edge (102);
    a rear waist portion (120) with a rear waist edge (122);
    a crotch portion (130) comprising a crotch gusset (230), the crotch portion having a forward portion (132) meeting the front waist portion and a rearward portion (136) meeting the rear waist portion; and
    left and right hip side portions joining the front waist portion to the rear waist portion and thereby forming a waist opening with a waist opening edge comprising the front waist edge and the rear waist edge, and left and right leg openings defined by respective left and right leg opening edges (104, 124, 140);
    wherein, when the pant is in an opened configuration in which the front waist portion and rear waist portion are separated at the hip side portions, the pant has a longitudinal axis (200) and a lateral axis (300), with an intersection (250) thereof, the intersection occurring in the crotch portion;
    wherein the crotch gusset (230) comprises an absorbent assembly (238) comprising an absorbent layer (232), a barrier layer (233) disposed beneath the absorbent layer; and an outward-facing layer (234) disposed beneath the barrier layer, the absorbent layer having respective left and right edges (231e) respectively proximate the left and right leg opening edges; and
    wherein, proximate each of the left and right leg opening edges, the absorbent assembly (238) comprises a liquid impermeable edge sealing structure comprising a liquid impermeable film (233, 235) and having an upper portion (235u, 233u) affixed to a wearer-facing surface of the absorbent assembly (238), and a lower portion (235L, 233L) beneath the absorbent layer (232), wherein the outward-facing layer (234) is disposed beneath the lower portion (235L, 233L), and wherein the upper portion (235u, 233u) of the edge sealing structure includes a portion disposed inwardly of any outwardly-lying adjacent wearer-facing fabric surface by a barrier margin (HM) having an average dimension greater than 0 mm, more preferably at least 1 mm, and even more preferably at least 2 mm;
    the pant further comprising a finishing material (237) proximate the leg opening edges (140) and having a first portion affixed to an upper portion of the edge sealing structure, and a second portion affixed to the outward-facing layer (234);
    wherein the absorbent assembly comprises a wearer-facing layer (231) comprising a first knitted fabric material, disposed above the absorbent layer (232), wherein the first knitted fabric material is knitted of one or more yarns that comprise synthetic material selected from the group consisting of polyester, polyamide, polypropylene, polyethylene and combinations thereof;
    wherein the crotch portion comprises four layers comprising a wearer-facing layer (231), an absorbent layer (232) directly beneath the wearer-facing layer, a liquid impermeable barrier layer (233) beneath the absorbent layer and an outward-facing layer (234) beneath the barrier layer;
    and
    wherein any of the wearer-facing layer, absorbent layer and outward-facing layer comprises elastic filaments.

2.  The pant of claim 1 wherein the liquid impermeable film (233, 235) is elastically extensible.

3.  The pant of any of the preceding claims wherein the crotch gusset (230) exhibits a maximum Longitudinal Elongation of 25 percent to 100 percent and a Longitudinal Tensile Modulus of 10 gf/mm to 100 gf/mm.

4.  The pant of any of the preceding claims wherein the crotch portion has a central Caliper at the intersection (250) of lateral and longitudinal axes, of no greater than 5 mm, more preferably no greater than 4 mm, and even more preferably no greater than 3.5 mm.

5.  The pant of any of the preceding claims wherein the absorbent layer (232) comprises a second knitted fabric material.

6.  The pant of claim 5 wherein the second knitted fabric material is a knitted terrycloth material.

7.  The pant of either of claims 5 or 6 wherein the second knitted fabric material is knitted of one or more yarns that comprise material selected from the group consisting of cotton, rayon, polyester, polyamide and combinations thereof.

8.  The pant of any of claims 5-7 wherein the second knitted fabric material comprises a microfiber yarn.

**Patentansprüche**

1. Strapazierfähiges Absorptionshöschen, umfassend:

einen vorderseitigen Taillenabschnitt (100) mit einem vorderseitigen Taillenrand (102);
einen rückseitigen Taillenabschnitt (120) mit einem rückseitigen Taillenrand (122);
einen Schrittabschnitt (130), umfassend einen Schrittzwickel (230), wobei der Schrittabschnitt einen vorderen Abschnitt (132), der auf den vorderseitigen Taillenabschnitt trifft, und einen hinteren Abschnitt (136), der auf den rückseitigen Taillenabschnitt trifft, aufweist; und
linke und rechte Hüftseitenabschnitte, die den vorderseitigen Taillenabschnitt mit dem rückseitigen Taillenabschnitt verbinden und dadurch eine Taillenöffnung mit einem Taillenöffnungsrand ausbilden, umfassend den vorderen Taillenrand und den rückseitigen Taillenrand, und linke und rechte Beinöffnungen, die durch jeweilige linke und rechte Beinöffnungsränder (104, 124, 140) definiert sind;
wobei, wenn das Höschen in einer geöffneten Konfiguration ist, in der der vorderseitige Taillenabschnitt und der rückseitige Taillenabschnitt an den Hüftseitenabschnitten getrennt sind, das Höschen eine Längsachse (200) und eine Querachse (300) mit einem Schnittpunkt (250) davon aufweist, wobei der Schnittpunkt in dem Schrittabschnitt auftritt;
wobei der Schrittzwickel (230) eine Absorptionseinheit (238) umfasst, umfassend eine Absorptionsschicht (232), eine unter der Absorptionsschicht angeordnete Sperrschicht (233); und eine nach außen gerichtete Schicht (234), die unter der Sperrschicht angeordnet ist, wobei die Absorptionsschicht jeweilige linke und eine rechte Ränder (231e) jeweils in der Nähe der linken und rechten Beinöffnungsränder aufweist; und
wobei die Absorptionseinheit (238) in der Nähe jedes der linken und rechten Beinöffnungsränder eine flüssigkeitsundurchlässige Randversiegelungsstruktur umfasst, umfassend eine flüssigkeitsundurchlässige Folie (233, 235), und die einen oberen Abschnitt (235u, 233u), der an einer trägerseitigen Oberfläche der Absorptionseinheit (238) befestigt ist, und einen unteren Abschnitt (235L, 233L) unter der Absorptionsschicht (232) aufweist, wobei die nach außen gerichtete Schicht (234) unter dem unteren Abschnitt (235L, 233L) angeordnet ist, und wobei der obere Abschnitt (235u, 233u) der Randversiegelungsstruktur einen Abschnitt einschließt, der innerhalb einer beliebigen nach außen liegenden, angrenzenden, trägerseitigen Stoffoberfläche durch einen Sperrspielraum (HM) angeordnet ist, der eine durchschnittliche Abmessung größer als 0 mm, mehr bevorzugt mindestens 1 mm und noch mehr bevorzugt mindestens 2 mm aufweist;
das Höschen ferner umfassend ein Endbearbeitungsmaterial (237) in der Nähe der Beinöffnungsränder (140) und das einen ersten Abschnitt, der an einem oberen Abschnitt der Randversiegelungsstruktur befestigt ist, und einen zweiten Abschnitt aufweist, der an der nach außen gerichteten Schicht (234) befestigt ist;
wobei die Absorptionseinheit eine trägerseitige Schicht (231) umfasst, umfassend ein erstes Maschenstoffmaterial, das über der Absorptionsschicht (232) angeordnet ist, wobei das erste Maschenstoffmaterial aus einem oder mehreren Garnen gestrickt ist, die synthetisches Material, ausgewählt aus der Gruppe bestehend aus Polyester, Polyamid, Polypropylen, Polyethylen und Kombinationen davon, umfassen;
wobei der Schrittabschnitt vier Schichten umfasst, umfassend eine trägerseitige Schicht (231), eine Absorptionsschicht (232) direkt unterhalb der trägerseitigen Schicht, eine flüssigkeitsundurchlässige Sperrschicht (233) unterhalb der Absorptionsschicht und eine nach außen gerichtete Schicht (234) unterhalb der Sperrschicht; und
wobei jede beliebige der trägerseitigen Schicht, der Absorptionsschicht und der nach außen gerichteten Schicht elastische Fäden umfasst.

2. Höschen nach Anspruch 1, wobei die flüssigkeitsundurchlässige Folie (233, 235) elastisch dehnbar ist.

3. Höschen nach einem der vorstehenden Ansprüche, wobei der Schrittzwickel (230) eine maximale Längsdehnung von 25 Prozent bis 100 Prozent und einen Längszugmodul von 10 gf/mm bis 100 gf/mm vorweist.

4. Höschen nach einem der vorstehenden Ansprüche, wobei der Schrittabschnitt an dem Schnittpunkt (250) der Quer- und Längsachsen eine zentrale Dicke von nicht mehr als 5 mm, mehr bevorzugt nicht mehr als 4 mm und noch mehr bevorzugt nicht mehr als 3,5 mm aufweist.

5. Höschen nach einem der vorstehenden Ansprüche, wobei die Absorptionsschicht (232) ein zweites Maschenstoffmaterial umfasst.

6. Höschen nach Anspruch 5, wobei das zweite Maschenstoffmaterial ein gestricktes Frotteematerial ist.

7. Höschen nach einem der Ansprüche 5 oder 6, wobei das zweite Maschenstoffmaterial aus einem oder mehreren Garnen gestrickt ist, die Material umfassen ausgewählt aus der Gruppe, bestehend aus Baumwolle, Rayon, Polyester, Polyamid und Kombinationen dav on.

**8.** Höschen nach einem der Ansprüche 5 bis 7, wobei das zweite Maschenstoffmaterial ein Mikrofasergarn umfasst.

## Revendications

**1.** Culotte absorbante durable, comprenant :

une région de ceinture avant (100) et un bord de ceinture avant (102) ;
une région de ceinture arrière (120) et un bord de ceinture arrière (122) ;
une partie d'entrejambe (130) comprenant un gousset d'entrejambe (230), la partie d'entrejambe ayant une partie avant (132) rejoignant la partie de ceinture avant et une partie arrière (136) rejoignant la partie de ceinture arrière ; et
des parties latérales de hanche gauche et droite reliant la partie de ceinture avant à la partie de ceinture arrière et formant de ce fait une ouverture de ceinture avec un bord d'ouverture de ceinture comprenant le bord de ceinture avant et le bord de ceinture arrière, et les ouvertures de jambe gauche et droite définies par des bords d'ouverture de jambe gauche et droite respectives (104, 124, 140) ;
dans laquelle, lorsque la culotte est dans une configuration ouverte dans laquelle la partie de ceinture avant et la partie de ceinture arrière sont séparées au niveau des parties latérales de hanche, la culotte a un axe longitudinal (200) et un axe latéral (300), avec une intersection (250) de ceux-ci, l'intersection se produisant dans la partie d'entrejambe (130) ;
dans lequel le gousset d'entrejambe (230) comprend un ensemble absorbant (238) composé d'une couche absorbante (232) et d'une couche barrière (233) disposée sous la couche absorbante ; et une couche orientée vers l'extérieur (234) disposée sous la couche barrière, la couche absorbante ayant des bords gauche et droit (231e) respectifs respectivement à proximité des bords d'ouverture de jambe gauche et droite ; et
dans lequel, à proximité de chacun des bords d'ouverture de jambe gauche et droite, l'ensemble absorbant (238) comprend une structure de scellement des bords imperméable aux liquides comprenant un film imperméable aux liquides (233, 235) et ayant une partie supérieure (235u, 233u) fixée à une surface de l'ensemble absorbant (238) orientée vers le porteur, et une partie inférieure (235L, 233L) sous la couche absorbante (232), dans laquelle la couche orientée vers l'extérieur (234) est disposée sous la partie inférieure (235L, 233L), et
dans laquelle la partie supérieure (235u, 233u) de la structure de scellement des bords comporte une partie disposée vers l'intérieur de toute surface de tissu adjacente orientée vers le porteur par une marge de barrière (HM) ayant une dimension moyenne supérieure à 0 mm, plus préférablement au moins 1 mm, et encore plus préférablement au moins 2 mm ;
la culotte comprend en outre un matériau de finition (237) situé à proximité des bords d'ouverture de la jambe (140) et ayant une première partie fixée à une partie supérieure de la structure de scellement des bords, et une seconde partie fixée à la couche orientée vers l'extérieur (234) ;
dans laquelle l'ensemble absorbant comprend une couche orientée vers le porteur (231) comprenant un matériau en tissu tricoté disposé au-dessus de la couche absorbante (232), dans laquelle le matériau en tissu tricoté est tricoté d'une ou plusieurs laines qui comprend un matériau synthétique choisi dans le groupe constitué de polyester, polyamide, polypropylène, polyéthylène et des combinaisons de ceux-ci ;
dans laquelle la partie d'entrejambe comprend quatre couches comprenant une couche orientée vers le porteur (231), une couche absorbante (232) directement sous la couche orientée vers le porteur, une couche barrière imperméable aux liquides (233) sous la couche absorbante et une couche faisant face vers l'extérieur (234) sous la couche barrière ; et
dans laquelle l'une quelconque de la couche orientée vers le porteur, de la couche absorbante et de la couche orientée vers l'extérieur comprend des filaments élastiques.

**2.** Culotte selon la revendication 1 dans lequel le film imperméable aux liquides (233, 235) est élastiquement extensible.

**3.** Culotte selon l'une quelconque des revendications précédentes dans laquelle le gousset d'entrejambe (230) présente un allongement longitudinal maximal de 25 % à 100 % et un module de traction longitudinal de 10 gf/mm à 100 gf/mm.

**4.** Culotte selon l'une quelconque des revendications précédentes dans laquelle la partie d'entrejambe a une épaisseur centrale à l'intersection (250) des axes latéraux et longitudinaux, ne dépassant pas 5 mm, de préférence ne dépassant pas 4 mm, et encore plus préférentiellement ne dépassant pas 3,5 mm.

5. Culotte selon l'une quelconque des revendications précédentes, dans lequel la couche absorbante (232) comprend un second matériau en tissu tricoté.

6. Culotte selon la revendication 5, dans lequel le second tissu tricoté est un tissu éponge tricoté.

7. Culotte selon l'une des revendications 5 ou 6, dans laquelle le second matériau en tissu tricoté est un matériau éponge tricoté, dans laquelle le matériau en tissu tricoté est tricoté d'une ou plusieurs laines qui comprennent un matériau choisi dans le groupe constitué de coton, rayonne, viscose, polyester, polyamide et des combinaisons de ceux-ci.

8. Culotte selon l'une quelconque des revendications 5 à 7, dans laquelle le second matériau en tissu tricoté comprend une laine de microfibre.

**FIG. 1**

**FIG. 2**

**FIG. 3A**

**FIG. 3B**

**FIG. 3C**

**FIG. 3D**

**FIG. 3E**

**FIG. 3F**

**FIG. 4**

FIG. 5A

FIG. 5B

FIG. 6

**FIG. 7A**

**FIG. 7B**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 110022816 B

- US 20170369698 A